# EUROPÄISCHE PATENTSCHRIFT

(11) **EP 2 435 394 B1**
(45) Veröffentlichungstag und Bekanntmachung des Hinweises auf die Patenterteilung: **08.05.2013**
(21) Anmeldenummer: 10727642.0
(22) Anmeldetag: 31.05.2010
(51) Int. Cl.: A61K 31/122, C07C 50/34, C07C 69/013, C12P 7/66, C12P 15/00

(54) **NEUE ANTHRACHINON-DERIVATE**
NOVEL ANTHRAQUINONE DERIVATIVES
NOUVEAUX DÉRIVÉS D'ANTRAQUINONE

(30) Priorität: 29.05.2009 AT 8422009
(43) Veröffentlichungstag der Anmeldung: 04.04.2012
(73) Patentinhaber: Sealife Pharma GmbH, 3430 Tulln (AT)
(72) Erfinder: PRETSCH, Alexander, 2002 Großmugl (AT); PROKSCH, Peter, 52428 Jülich (DE); DEBBAB, Abdessamad, 40215 Düsseldorf (DE)
(74) Vertreter: Ellmeyer, Wolfgang
(86) Internationale Anmeldenummer: PCT/AT2010/000190
(87) Internationale Veröffentlichungsnummer: WO 2010/135759

(56) Entgegenhaltungen:
- DE-A1- 4 121 468
- OKAMURA N ET AL: "Altersolanol-related antimicrobial compounds from a strain of Alternaria solani" PHYTOCHEMISTRY, Bd. 34, Nr. 4, 1. November 1993 (1993-11-01), Seiten 1005-1009, XP026631985 [gefunden am 1993-11-01] in der Anmeldung erwähnt
- BUCHANAN MALCOLM S ET AL: "Pigments of fungi. XLVI. New dimeric dihydroanthracenones of the flavomannin type from an Australian toadstool of the genus Dermocybe" AUSTRALIAN JOURNAL OF CHEMISTRY, Bd. 51, Nr. 2, 1. Januar 1998 (1998-01-01) , Seiten 103-109, XP009139105

## Beschreibung

Die vorliegende Erfindung betrifft neue Anthrachinon-Derivate, ein Verfahren zu deren Herstellung sowie deren Verwendung als Antiinfektiva, vor allem gegen mehrfach wirkstoffresistente Erreger, sowie als Antikrebsmittel.

### STAND DER TECHNIK

Durch den höchst erfolgreichem Einsatz antimikrobieller Arzneimittel wurde in den späten 1960er- und frühen 1970er-Jahren angenommen, dass Infektionskrankheiten fortan keine Gefahr mehr darstellen würden. Dies sollte sich als schwerer Irrtum herausstellen, zumal die Mikroben 40 Jahre danach eine größere Bedrohung als jemals zuvor darstellen, weswegen ein dringender Bedarf an neuen antimikrobiellen Wirkstoffen besteht. Heutzutage sind Infektionskrankheiten die dritthäufigste Todesursache in den USA und die zweithäufigste weltweit. Für die meisten dieser Fälle sind unwirksame antimikrobielle Arzneimittel verantwortlich, und die Resistenz mancher Bakterien und Pilze gegen diese Wirkstoffe stellt in unserer Gesellschaft ein ernstes Problem dar. Statistischen Daten aus den USA zufolge wird der Großteil der im Krankenhaus erlittenen Infektionen (den sog. nosokomialen Infektionen) von wenigen Bakterienspezies hervorgerufen, nämlich von *Enterococcus faecium, Staphylococcus. aureus, Klebsiella pneumoniae, Acinetobacter baumanii, Pseudomonas aeruginosa* und *Enterobacter sp.,* die nach ihren Anfangsbuchstaben als "ESKAPE"-Pathogene bezeichnet werden (Boucher et al., "IDSA Report on Development Pipeline", CID 2009:48, Infectious Disease Society of America, 1. Januar 2009). Mit diesem Begriff soll freilich gleichzeitig ausgedrückt werden, dass sich diese resistenten Erreger der Wirkung antibakterieller Arzneimittel entziehen (engl.: *to escape),* zumal Antibiotikaresistenz auf molekularer Ebene nichts anderes bedeutet als, dass ein Mikroorganismus die Fähigkeit erlangt hat, sich der wachstumshemmenden oder bakteriziden Wirkung einer antimikrobiellen Substanz zu widersetzen. Das heißt, die Substanz wird klinisch unwirksam. So bedeutet etwa "methicillinresistenter *Staphylococcus aureus"* (auch als MRSA abgekürzt, wiewohl dieselbe Abkürzung auch in allgemeinerer Weise für "multiresistenten *Staphylococcus aureus"* gebraucht wird), dass die Verwendung von β-Lactamen in der Therapie von *S. aureus* unwirksam ist, während beispielsweise Glykopeptide zumeist Wirkung zeigen. Es ist daher unbedingt erforderlich die Wirkung von neuen Antiinfektiva gegen multiresistente Stämmen zu testen, da Wirksamkeit gegen dafür empfindliche Spezies oder Stämme nicht zwangsläufig auch Wirksamkeit gegen multiresistente Bakterien bedeutet, wie auch beispielsweise ein gegen grampositive Bakterien wirksames Mittel nicht notwendigerweise gegen gramnegative Bakterien wirkt oder umgekehrt (siehe u.a. Boucher et al., s.o.).

Neben Bakterien und Pilzen fehlen gegenwärtig aber auch wirksame Strategien gegen respiratorische Viren. Zumeist werden lediglich die Symptome geheilt, ohne das Virus selbst zu bekämpfen. Eine Lösung für die Zukunft könnten vor allem Wirkstoffkombinationen sein.

In der Vergangenheit wurden in endophytischen Pilzen zahlreiche Substanzen zur Bekämpfung der Vermehrung von Mikroorganismen gefunden. Diese Substanzen zeigen in einer Reihe von Fällen sehr gute antibakterielle, antifungale und antivirale Aktivität und könnten für zahlreiche Anwendungen eingesetzt werden (G. A. Strobel, Crit. Rev. Biotechnol. 22, 315-333 (2002)). Aufgrund der Tatsache, dass diesbezügliche Forschung überwiegend akademisch war und nicht direkt die Entwicklung neuer Wirkstoffe zum Ziel hatte, sind heute kaum entsprechende Arzneimittel auf dem Markt. Insbesondere das Screenen gegen resistente Mikroben wurde in der Vergangenheit vernachlässigt, so dass nur einige wenige Substanzen gegen arzneimittelresistente Mikroben gestestet wurden. Noch schlimmer ist die Lage bei respiratorischen Viren, gegen die bisher nahezu keine Substanzen gescreent wurden.

Bereits im Jahre 1969 beschrieb A. Stoessl, Can. J. Chem. 47, 767 (1969), die Isolierung eines neuen metabolischen Pigments aus *Alternaria solani,* einem Schimmelpilz, der die sog. Dürrfleckenkrankheit bei Kartoffeln hervorruft. Dieses Pigment wurde Altersolanol A genannt und seine Struktur wie folgt indentifiziert:

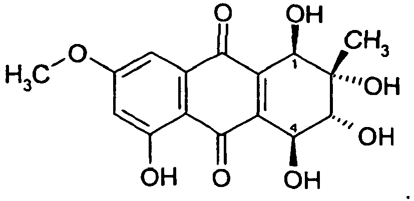

Der chemische Name dieses Anthrachinons lautet demnach 7-Methoxy-2-methyl-(1*R*,2*S*,3*R*,4*S*)-1,2,3,4-tetrahydro-1,2,3,4,5-pentahydroxyanthracen-9,10-dion.

In weiterer Folge wurde dieses Pigment sowie mehrere Isomere und Derivate davon auch in weiteren *Alternaria-*Spezies (z.B. *Alternaria porri)* und in manchen anderen Pilzgattungen nachgewiesen (siehe beispielsweise R. Suemitsu et al., Agric. Biol. Chem. 45(10), 2363-2364 (1981)). Die Mehrzahl der Isomere und Derivate entspricht den folgenden allgemeinen Formeln (1) oder (2): 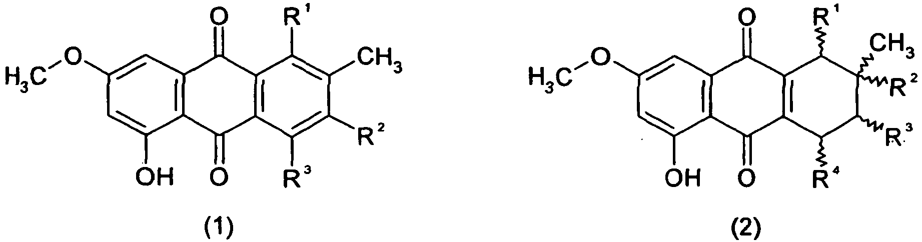 wobei R¹ bis R⁴ jeweils H oder OH sein können, was im Falle von OH in Formel (2) jeweils ein chirales Kohlenstoffatom ergibt, das sowohl in R- als auch in S-Konfiguration vorlieben kann.

In weiterer Folge wurden auch Dimere, d.h. Bisanthrachinone, als Metaboliten entdeckt, und das erstmals in *Alternaria porri,* einem weiteren Vertreter der Gattung der *Alternariae,* der u.a. die Purpurtleckenkrankheit bei Zwiebelgemüse hervorruft, weswegen diese Dimere Alterporriole genannt wurden. Sie entsprechen beispielsweise der folgenden Formel (3): 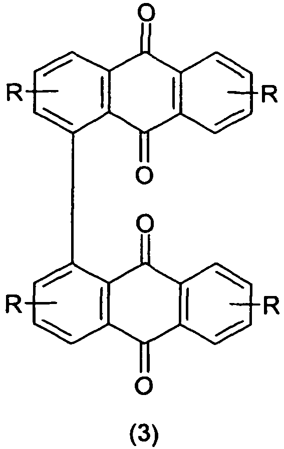 wobei ähnlich vielfältige Substitutions- und Hydrierungsmuster an den aromatischen Ringen möglich sind wie für die "monomeren" Altersolanole. Aufgrund eingeschränkter Freiheit der Rotation um die Achse der chemischen Bindung zwischen den Anthrachinon-Kemen liegen zahlreiche Alterporriol-Derivate in Form zweier Atropisomere vor.

Für manche als Altersolanole bzw. Alterporriole bezeichnete Verbindungen existieren Berichte über ihre Wirksamkeit gegen bestimmte Mikroorganismen, während andere als nicht antiinfektiv beschrieben wurden. So wurden etwa von Aly et al., Phytochemistry 69, 1716-1725 (2009), bioaktive Metaboliten endophytischer Pilze der Gattung *Ampelomyces* sowie deren antiinfektive Wirkung untersucht. Dabei wurde unter anderem festgestellt, dass Altersolanol J, die Alterporriole D und E (Atropisomere), sowie die mit Altersolanolen eng verwandte Verbindung Ampelanol keine Aktivität gegen Bakterien und Pilze zeigen, während Altersolanol A die wirksamste der getesteten Substanzen war. Weiters offenbaren beispielsweise Okamura et al., Phytochemistry 42(1), 77-80 (1996), keinerlei Wirkung von Tetrahydroaltersolanol B gegen grampositive Bakterien und die gramnegative Spezies *Pseudomonas aeruginosa.* Und Yagi et al., Phytochemistry 34(4), 1005-1009 (1993), berichten über die antimikrobielle Aktivität der Altersolanole A, B, C und E, während die Altersolanole D, E und F in demselben Test völlig unwirksam waren. In der US-Patentanmeldung Nr. 2007/258913 werden die atropisomeren Altersolanole D und E hingegen, wenn auch nur ganz allgemein, als geeignete Verbindungen zur Verhinderung der Bildung eines Biofilms in der Mundhöhle offenbart, ohne allerdings konkrete Daten bezüglich ihrer Wirksamkeit anzugeben.

Somit ist es jedenfalls unmöglich vorherzusagen, ob ein bestimmtes Altersolanol- bzw. Alterporriol-Isomer oder -Derivat antiinfektive Wirkung zeigt oder nicht, geschweige denn gegen welche Gattungen oder gar Spezies von Mikroorganismen.

Seit einigen Jahren zeigen sich auch bei Antikrebswirkstoffen ähnliche Tendenzen wie im Bereich der Antiinfektiva, nämlich eine stetige Zunahme von Resistenzen gegen bestehende Therapien. Hier beruht die Resistenzentwicklung vor allem darauf, dass eine Überexpression von integralen Membrantransportem, wie z.B. P-gp, zu einem Efflux von Wirkstoffen aus der Krebszelle führt, wodurch diese ihre Wirkung nicht mehr entfalten können. Solche multiresistente ("multiple drug resistant", MDR-) Zellen weisen mittlerweile Resistenzen gegen zahlreiche strukturell und mechanistisch unterschiedliche Chemotherapeutika auf (siehe etwa E.L. Cooper, Evid. Based Complement. Alternat. Med. 1, 215-217 (2004); M. D'lncalci et al., J. Chemother. 16 (Suppl. 4), 86-89 (2004); Z. Shi et al., Cancer Res. 67, 11012-11020 (2007)). Somit ist neben der Entwicklung neuer antiinfektiver Substanzen gegen pathogene Erreger auch die Entdeckung und Anwendung neuer Wirkstoffe im Krebsbereich zur Unterdrückung von Resistenzbildung im Körper wünschenswert.

Seit mehreren Jahrzehnten werden unter anderem auch Anthrachinone als wirksame Chemotherapeutika in der Krebstherapie eingesetzt. Bekannte Medikamente aus dieser Gruppe sind die strukturell sehr ähnlichen Wirkstoffe Doxorubicin (das nachstehend beispielhaft dargestellt ist), Daunorubicin, Idarubicin und Epirubicin, die gegen eine Vielzahl von Krebsarten eingesetzt werden.

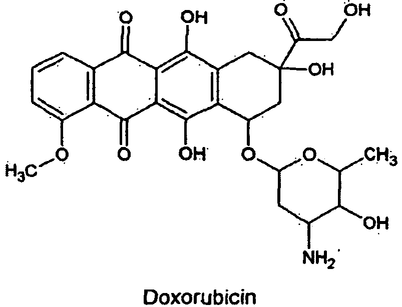

Derartige Wirkstoffe zeigen neben ihrer erwünschten therapeutischen Wirkung aber auch den negativen Effekt der Superoxid-Radikalbildung. Aufgrund der radikalstabilisierenden Wirkung der Chinongruppe im Molekül ist eine derartige Radikalbildung bei Anthrachinonen häufig anzutreffen und kann bei therapeutischer Verwendung solcher Moleküle unter anderem Kardiotoxizität hervorrufen. Sowohl die therapeutische als auch die radikalstabilisierende Wirkung der Chinongruppe werden dabei maßgeblich durch die Substituenten beeinflusst. Siehe beispielsweise die Untersuchungen mit Maus-Lymphom-Zellen von Debbab et al., J. Nat. Prod. 72(4), 626-631 (2009).

Als Wirkmechanismus der zytotoxischen Wirkung von Anthrachinonen wird vorwiegend eine Interkalation des Moleküls in die Doppelhelix der DNA angenommen, wodurch sowohl die Replikation als auch die Transkription der DNA zu RNA nicht mehr ordnungsgemäß ablaufen können. In Kombination damit dürften Anthrachinone auch Topoisomerase Typ II hemmen, die für die Superspiralisierung der DNA mitverantwortlich ist, und gelegentlich Apoptose auslösen. Ob ein Anthrachinon- oder sonstiges, ähnlich wirkendes - d.h. interkalierendes - Molekül erfolgreich in die Doppelhelix eingeschoben werden kann, hängt, wie oben erwähnt, maßgeblich von der Art und Lage der Substituenten ab. Wie aktuelle Untersuchungen mit verschiedenen Anthrachinon-Derivaten zeigen (siehe z.B. J. Zhang et al., Mar. Drugs 8, 1469-1481 (2010)), entscheiden äußerst geringfügige Unterschiede zwischen den Ringsubstituenten über die zytotoxische Aktivität der Moleküle.

Das Ziel der Erfindung war vor diesem Hintergrund die Identifikation, Isolierung und Herstellung neuer Substanzen zur Verwendung als antiinfektive Wirkstoffe bzw. als Antikrebsmittel in pharmazeutischen Zusammensetzungen, vor allem von Verbindungen, die Aktivität gegen mehrfach wirkstoffresistente ("multiple drug resistant", MDR-) Erreger bzw. Zellen zeigen.

Die Erfinder konnten nun im Verlauf ihrer Forschungen mehrere Substanzen - zum Teil mit bisher unveröffentlichter Struktur, d.h. neue chemische Verbindungen - identifizieren, die gute Aktivität gegen Mikroorganismen und respiratorische Viren, insbesondere gegen MDR-Erreger, aber auch gegen Krebszellen zeigen. Während in der parallelen, gleichzeitig eingereichten österreichischen Patentanmeldung mit der Anmeldenummer A 843/09 die Verwendung mehrerer Verbindungen mit bekannter Struktur, aber bislang unbekannter Wirksamkeit verfolgt werden, bezieht sich die vorliegende Erfindung auf die Bereitstellung zweier neuer Verbindungen, nämlich jeweils eines Altersolanol- und eines Alterporriol-Derivats.

### OFFENBARUNG DER ERFINDUNG

Konkret haben die Erfinder die folgenden neuen chemischen Verbindungen hergestellt, isoliert und charakterisiert:

(1*R*,2*S*,3*S*,4*R*)-3-Acetoxy-1, 2,4,5-tetrahydroxy-7-methoxy-2-methyl-1,2,3,4-tetrahydroanthracen-9,10-dion der Formel (4), das von den Erfindern ursprünglich als "Altersolanol M" bezeichnet worden war, mittlerweile jedoch zu "3-O-Acetyl-Altersolanol M" umbenannt wurde (da es international üblich ist, die nichtacetylierten Formen mit dem kennzeichnenden Buchstaben zu bezeichnen): 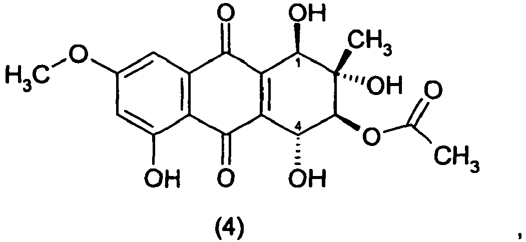 sowie 8-(4,5,6-Trihydroxy-7-methyl-2-methoxy-9,10-dioxo-9H,10H-anthracen-1-yl)-(1*S*,2*S*,3*R*,4*S*)-1,2,3,4,5-pentahydroxy-7-methoxy-2-methyl-1,2,3,4-tetrahydro-9H,10H-anthracen-9,10-dion der Formel (5), das in Form zweier Atropisomere existiert, die von den Erfindern als Alterporriole I und J bezeichnet wurden:

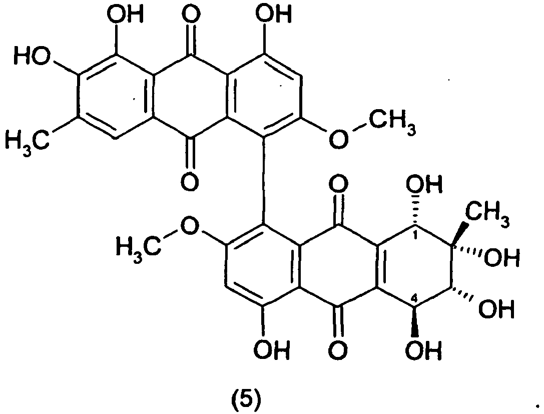

Da beide Verbindungen (4) und (5) in Screening-Versuchen ausgezeichnete antimikrobielle, antivirale und auch - speziell im Fall von Verbindung (4) - exzellente Antikrebs-Wirkung zeigen, besteht ein zweiter Aspekt der Erfindung in der Verwendung dieser neuen Verbindungen als Antiinfektiva, vorzugsweise gegen grampositive und gramnegative Bakterien, Pilze und respiratorische Viren, insbesondere als Antiinfektiva gegen mehrfach wirkstoffresistente ("multiple drug resistant", MDR-) Erreger, sowie in der Verwendung als Antikrebsmittel.

Speziell werden beide Verbindungen vorzugsweise gegen mehrfach wirkstoffresistente Stämme von methicillinresistentem *Staphylococcus aureus* (MRSA), *Staphylococcus epidermis, Streptococcus pneumoniae, Enterococcus faecalis* bzw. *faecium, Escherichia coli, Klebsiella sp., Pseudomonas aeruginosa, Aspergillus sp.* sowie gegen respiratorische Viren aus der Gruppe der humanen Rhinoviren und der respiratorischen Synzytial-Viren eingesetzt, was durch die späteren Ausführungsbeispiele im Detail belegt wird.

Weitere acetylierte Derivate von Altersolanol M, nämlich sowohl Isomere von 3-O-Acetyl-Altersolanol M mit der Acetylgruppe an anderer Position, wie z.B. 4-O-Acetyl-Altersolanol M und 5-O-Acetyl-Altersolanol M, und Derivate mit mehreren Acetylgruppen, z.B. 3,4-Di-O-acetyl- und 3,5-Di-O-acetyl-Altersolanol M, wurden in Vorversuchen ebenfalls auf ihre antiinfektive bzw. zytotoxische Wirkung getestet und lieferten zum Teil ebenfalls ausgezeichnete Ergebnisse, so dass die Herstellung und Untersuchung dieser Verbindungen Ziel von weiterführenden Forschungsarbeiten der Erfinder sein wird.

Die vorliegende Erfindung betrifft auch ein Verfahren zur Herstellung der Verbindungen der Formeln (4) und (5), das darin besteht, einen die Verbindung oder einen Vorläufer davon produzierenden Mikroorganismus unter Wachstumsbedingungen zu fermentieren und die jeweilige Verbindung, gegebenenfalls nach Zerstörung der Zellen des Mikroorganismus zur Erhöhung der Ausbeute, aus der Kultur zu gewinnen. In einem solchen Fermentationsverfahren wird als Mikroorganismus vorzugsweise ein reiner Stamm von *Stemphylium globuliferum* eingesetzt, da die Erfinder mit dieser Spezies die besten Ausbeuten erzielen konnten. Alternativ dazu können jedoch auch beliebige andere Mikroorganismen, die in der Lage sind, die neuen Verbindungen - oder auch Vorstufen davon - zu produzieren, z.B. *Alternaria sp.,* zur Fermentation eingesetzt werden. Werden durch die Fermentation Vorstufen erhalten, können diese nach beliebigen, dem einschlägigen Fachmann auf dem Gebiet der organischen Synthese wohlbekannten Verfahren in die gewünschten neuen Verbindungen der Formeln (4) und (5) übergeführt werden, wobei gegebenenfalls auch Verfahrensschritte unter Enzymkatalyse eingesetzt werden können, um höhere Enantioselektivität zu gewährleisten.

Beispielsweise kann etwa *Alternaria sp.* kultiviert werden, um aus der Fermentationsbrühe Altersolanol A - oder auch ein anderes Mitglied der Altersolanol-Familie - zu erhalten, das durch Acetylierung der OH-Gruppe an C3 (gegebenenfalls unter Schützen der übrigen OH-Gruppen mit herkömmlichen Schutzgruppen) in 3-O-Acetyl-Altersolanol M oder ein Stereoisomer davon übergeführt werden kann. Ein weiterer Syntheseweg besteht in der Abspaltung der OH-Gruppe an C3 oder C4 zusammen mit dem benachbarten Wasserstoffatom, d.h. Dehydratisierung, um eine Doppelbindung zwischen C3 und C4 und zwei prochirale Zentren an diesen Positionen zu erhalten. Anschließend kann diese Doppelbindung enzymatisch rehydratisiert werden, was bei geeigneter Wahl der Stereospezifität des Enzyms (z.B. Hydratase, Peroxygenase) das gewünschte Altersolanol M ergibt. Die Alterporriole I und J können beispielsweise auch durch chemische (z.B. enzymatische) Anbindung des entsprechenden Anthrachinon-Substituenten an Position 8 von Altersolanol M erhalten werden, das gegebenenfalls davor geeignet derivatisiert wurde, wonach die Acetylgruppe von der OH-Gruppe an Position 3 hydrolytisch abgespalten wird.

Geeignete Enzyme für die Syntheseschritte zur Umsetzung von Vorläufern der gewünschten Verbindung können in manchen Fällen ebenfalls aus dem zur Fermentation eingesetzten oder auch aus einem anderen Mikroorganismus isoliert werden. Letzterer Fall kann sinnvoll sein, wenn beispielsweise ein Mikroorganismus zwar das gewünschte Produkt produziert, aber z.B. in so geringen Mengen oder so stark verunreinigt, dass es wirtschaftlicher ist, durch Kultivierung eines anderen Stamms (oder sogar einer anderen Spezies oder Gattung) einen Vorläufer des Produkts zu erhalten und diesen mittels enzymatischer Synthese zur Zielverbindung umzusetzen.

Die Erfindung betrifft schließlich auch Verfahren zur Herstellung von 3-O-Acetyl-Altersolanol M, d.h. Verbindung (4) der vorliegenden Erfindung, durch chemische Synthese nach einem der folgenden Synthesewege A bis C.

### A) Mittels syn-Dihydroxylierung

Ausgehend von (1*R*,2*R*)-1,2,5-Trihydroxy-7-methoxy-2-methyl-1,2-dihydroanthracen-9,10-dion (6), das auf literaturbekannte Weise erhältlich ist (Krohn et al., Liebigs Ann. Chem. 1988, 1033-1041), kann 3-O-Acetyl-Altersolanol M durch folgende Schritte erhalten werden:
a) *syn*-Dihydroxylierung von (6) an C3 und C4 (gemäß Houben-Weyl, Handbuch der organischen Chemie, Band E21d, "Stereoselektive Synthese", S. 4581-4587) zu (1*R*,2*S*,3*R*,4*R*)-3-Acetoxy-1,2,4,5-tetrahydroxy-7-methoxy-2-methyl-1,2,3,4-tetrahydroanthracen-9,10-dion (7): 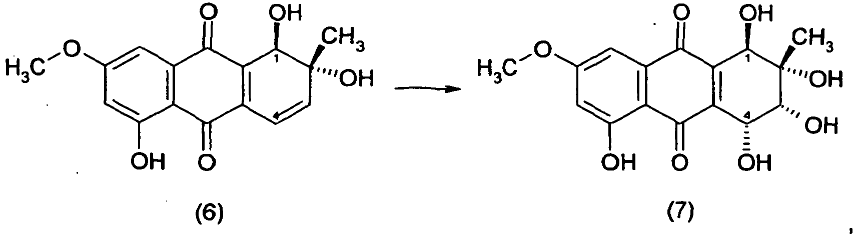
b) Überführung von (7) (gemäß Y. Gao und K.B. Sharpless, J. Am. Chem. Soc. 110, 7538-7539 (1988)) in das zyklische Sulfat (8): 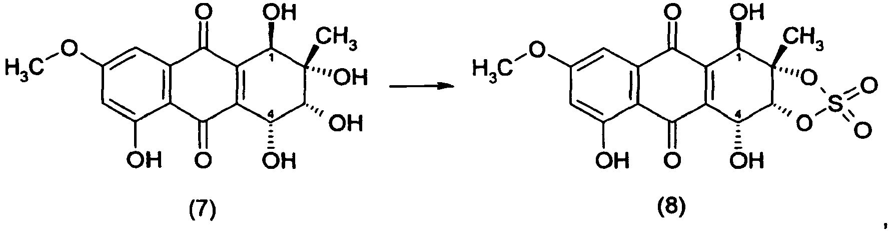
c) Acidolyse des zyklische Sulfats (8) (gemäß H.-S. Buyn et al., Tetrahedron 56, 7051-7091 (2000)) mit Essigsäure zu 3-O-Acetyl-Altersolanol M der Formel (4):

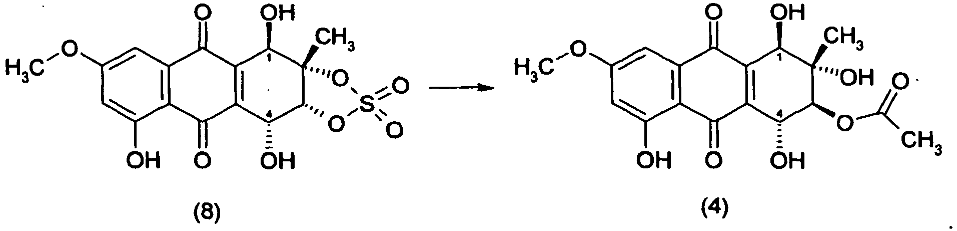

Gegenüber dem bei Krohn et al. (s.o.) beschriebenen Verfahren zur Herstellung von (unter anderem) Altersolanol A und Altersolanol M wird nach dem erfindungsgemäßen Verfahren die Doppelbindung von Verbindung (6) zwischen C3 und C4 nicht mit meta-Chlorperbenzoesäure zum Epoxid oxidiert, da dieser Schritt zwangsläufig ein Diastereomerengemisch aus cis- und trans-Epoxyalkohol ergibt, was die Ausbeute am gewünschten Isomer drastisch verringert. Durch die erfindungsgemäße syn-Dihydroxylierung in Schritt a) kann selektiv das gewünschte Isomer erhalten werden, das durch einfache Acidolyse mit Essigsäure in die Zielverbindung 3-O-Acetyl-Altersolanol M übergeführt werden kann.

### B) Mittels Diels-Alder-Addition und Epoxidbildung

Ausgehend von geschütztem 5-Hydroxy-7-methoxy-1,4-naphthochinon (9), das auf literaturbekannte Weise erhältlich ist (Krohn et al., Liebigs Ann. Chem. 1988, 1033-1041), kann 3-O-Acetyl-Altersolanol M durch folgende Schritte erhalten werden:
a) Diels-Alder-Addition zwischen Naphthochinon (9) und Methylbutadien (10), worin die R unabhängig voneinander gleiche oder unterschiedliche Hydroxyl-Schutzgruppen darstellen, zum Tetrahydroanthrachinon (11): 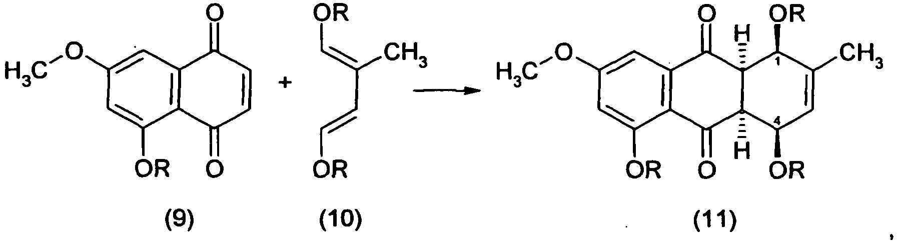
b) Oxidation der Doppelbindung zwischen C2 und C3 mit meta-Chlorperbenzoesäure (gemäß Krohn et al. (s.o.), unter anschließender oder vorhergehender Mitsunobu-Reaktion zur Inversion der Sauerstoff-Funktionalität an C4, zum Epoxid (12): 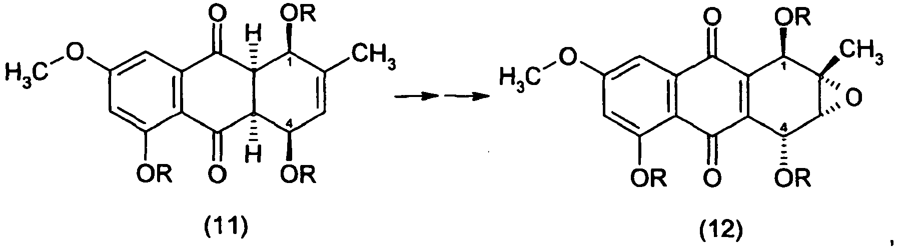
c) Acidolyse des Epoxids (12) mit Essigsäure unter anschließender oder gleichzeitiger Abspaltung der Schutzgruppen R zu 3-O-Acetyl-Altersolanol M.

Im Unterschied zu der von Krohn et al. beschriebenen Diels-Alder-Reaktion wird gemäß vorliegender Erfindung bereits ein Butadien (10) mit zwei Sauerstoff-Funktionalitäten, d.h. geschützten Hydroxylgruppen, eingesetzt, die diastereoselektiv gleichzeitig an C1 und C4 in das Anthrachinon (11) eingebaut werden, das in nur einem einzigen Oxidationsschritt stereoselektiv in das Epoxid (12) überführbar ist, da die Substituenten an C1 und C2 dirigierende Wirkung auf die Oxidation ausüben. Vorzugsweise erfolgt daher auch die Mitsunobu-Reaktion zur Inversion der Sauerstoff-Funktionalität an C4 erst nach der Oxidation. Die auf Letztere folgende Acidolyse läuft ebenfalls stereoselektiv ab, da der nucleophile Angriff des Acetats an C3 von der dem Epoxid-Sauerstoff gegenüberliegenden Seite klar begünstigt ist.

### C) Mittels Diels-Alder-Addition und syn-Dihydroxylierung

Wiederum ausgehend vom geschützten 5-Hydroxy-7-methoxy-1,4-naphthochinon (9) kann 3-O-Acetyl-Altersolanol M auch durch folgende Schritte erhalten werden:
a) Diels-Alder-Addition zwischen Naphthochinon (9) und Methylbutadien (10), worin die R unabhängig voneinander gleiche oder unterschiedliche Hydroxyl-Schutzgruppen darstellen, zum Tetrahydroanthrachinon (11), wie zuvor in Verfahren B) beschrieben: 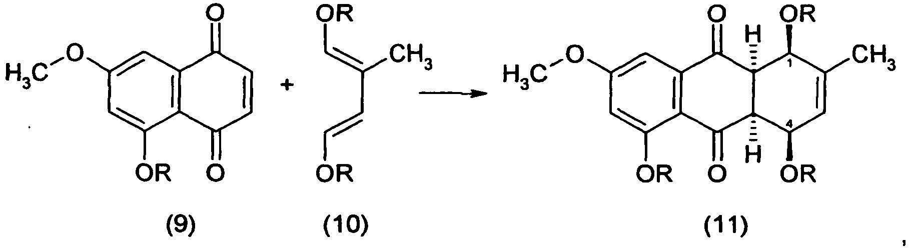
b) *syn*-Dihydroxylierung des Tetrahydroanthrachinons (11) (gemäß Houben-Weyl, s.o.), unter anschließender oder vorhergehender Mitsunobu-Reaktion zur Inversion der Sauerstoff-Funktionalität an C4, zum Tetrahydroanthrachinon (13): 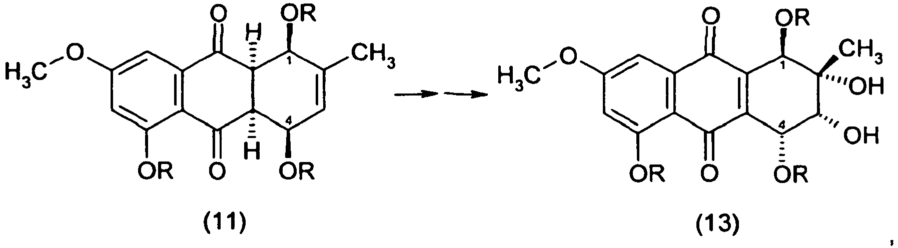
c) Überführung von (13) in das zyklische Sulfat (14) (gemäß Y. Gao und K.B. Sharpless, s.o.): 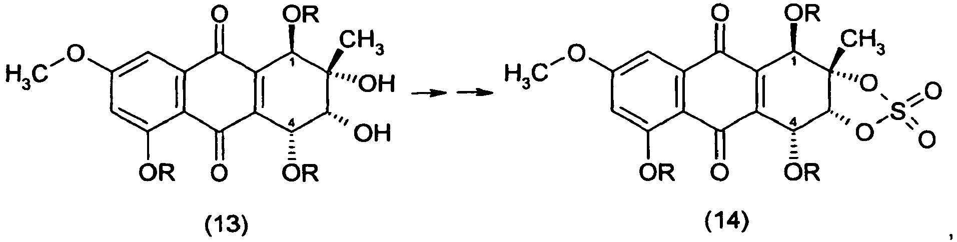
d) Acidolyse des zyklischen Sulfats (14) mit Essigsäure (gemäß H.-S. Buyn et al., s.o.), unter anschließender oder gleichzeitiger Abspaltung der Schutzgruppen R, zu 3-O-Acetyl-Altersolanol M.

Zum Unterschied zu Verfahren B) wird also das Tetrahydroanthrachinon (11) nicht zu einem Epoxid, sondern diastereoselektiv zum Dihydroxy-Derivat (13) oxidiert, das wie in Verfahren A) leicht in ein zyklisches Sulfat und anschließend in 3-O-Acetyl-Altersolanol M überführbar ist.

Anstelle zyklischer Sulfate sind die obigen Diole auch in andere zyklische Ester, so z.B. auch zyklische Orthoester überführbar, die ebenfalls im Schutzumfang der erfindungsgemäßen Verfahren liegen sollen.

Sobald die Erfinder die gegenwärtig laufenden Versuche zur Ausbeuteoptimierung abgeschlossen haben, wird die chemische Synthese aufgrund der höheren Reinheit bzw. einfacheren Isolierbarkeit der dabei erhaltenen Produkte im Vergleich zu Fermentationsverfahren die Methode der Wahl zur Herstellung von 3-O-Acetyl-Altersolanol M, aber auch von Alterporriol I und J sein. Eine Synthesestrategie für die Herstellung dieser Verbindungen der Formel (5) wird von den Erfindern zurzeit entwickelt.

Derzeit erfolgt die Gewinnung der jeweiligen Verbindung (4) oder (5) gemäß vorliegender Erfindung jedoch noch vorzugsweise durch Extraktion und anschließende Isolierung aus einem Rohextrakt einer Kultur, z.B. mittels fraktionierter Kristallisation oder Chromatographieverfahren, noch bevorzugter präparativer HPLC, was bisher die besten Ausbeuten bei gleichzeitig höchster Reinheit ergeben hat. Freilich sind speziell bei Ansätzen in größerem Maßstab auch andere Isolierungsverfahren, wie z.B. direkte fraktionierte Kristallisation des Rohextrakts oder auch Absorptionsmethoden, denkbar. Der Fachmann kann jedoch ohne übermäßiges Experimentieren das für den jeweiligen Kultivierungs- (bzw. Synthese- oder Partialsynthese-) Schritt geeignete Isolierungsverfahren ermitteln.

### BEISPIELE

Die Erfindung wird nachstehend unter Bezugnahme auf konkrete Ausführungsbeispiele näher beschrieben, die jedoch den Schutzumfang nicht einschränken sollen.

Die neuen Verbindungen der Erfindung wurden durch Kultivierung von *Stemphylium globuliferum* und anschließende Extraktion erhalten.

### Isolierung des Mikroorganismus

Frische, gesunde Stängel von *Mentha pulegium* (Polei-Minze) wurden zur Isolierung des endophytischen Pilzes verwendet. Die Oberfläche der Stängel wurde mit 70%-igem Ethanol 1 min lang sterilisiert und danach mit sterilem Wasser abgespült, um den Alkohol zu entfernen. Zur Unterscheidung etwaiger verbliebener epiphytischer Pilze von endophytischen Pilzen wurde ein Abdruck der Stängeloberfläche auf Biomalzagar angefertigt. Kleine Gewebeproben aus dem Inneren wurden aseptisch aufgeschnitten und auf Agarplatten gepresst, die zur Unterdrückung von bakteriellem Wachstum ein Antibiotikum enthielten. Die Zusammensetzung des Isoliermediums war folgende: 15 g/l Malzextrakt, 15 g/l Agar und 0,2 g/l Chloramphenicol in destilliertem Wasser, pH 7,4-7,8). Aus den Kulturen wurde durch wiederholtes Überimpfen auf Malzagarplatten ein reiner Pilzstamm erhalten.

Die Pilzkultur wurde nach einer molekularbiologischen Vorschrift mittels DNA-Amplifikation und Sequenzierung der ITS-Region als *Stemphylium globuliferum* identifiziert. Die Sequenzdaten wurden bei GenBank unter der Zugriffsnummer EU859960 hinterlegt.

### Kultivierung

Zur Kultivierung von *Stemphylium globuliferum* wurden zwei 1-Liter-Erlenmeyer-Kolben, die jeweils 100 g Reis und 100 ml destilliertes Wasser enthielten, autoklaviert, um ein gequollenes, festes Reismedium zu erhalten. Ein kleiner Teil des obigen Isoliermediums, das den reinen Pilzstamm enthielt, wurde unter sterilen Bedingungen auf das feste Reismedium aufgebracht, und der Pilz wurde bei Raumtemperatur (22 °C) 40 d lang kultiviert.

### Extraktion und Isolierung

Nach 40 d wurde die Kultur zweimal mit 300 ml Ethylacetat (EtOAc) extrahiert. Der EtOAc-Extrakt wurde mit Wasser ausgeschüttelt. Die wässrige Phase wurde bis zur Trockene eingedampft, und der Rückstand wurde zunächst Säulenchromatographie über eine Sephadex LH-20-Säule mit 100%igem Methanol (MeOH) als Laufmittel unter Detektion mittels Dünnschichtchromatographie (DC) auf Kieselgel F245 (Merck, Darmstadt, Deutschland) unter Verwendung von EtOAc/MeOH/H₂O (77:13:10) als Laufmittel unterzogen. Die die gewünschten Verbindungen enthaltenden Fraktionen wurden vereinigt und semipräparativer HPLC (Merck, Hitachi L-7100) unter Verwendung einer Eurosphere 100-10 C18-Säule (300 x 8 mm, L x i.d.) und eines linearen Wasser-Methanol-Gradienten unterzogen. Auf diese Weise wurden die neuen Verbindungen der Erfindung, d.h. (1*R*,2*S*,3*S*,4*R*)-3-Acetoxy-1,2,4,5-tetrahydroxy-7-methoxy-2-methyl-1,2,3,4-tetrahydroanthracen-9,10-dion (3-O-Acetyl-Altersolanol M) und 8-(4,5,6-Trihydroxy-7-methyt-2-methoxy-9,10-dioxo-9H,10H-anthracen-1-yl)-(1*S*,2*S,* 3*R*,4*S*)-1,2,3,4,5-pentahydroxy-7-methoxy-2-methyl-1,2,3,4-tetrahydro-9H,10H-anthracen-9,10-dion (Atropisomere Alterporriol I und J) in reiner Form erhalten, wobei die Atropisomere Alterporriol I und J jedoch nicht getrennt werden konnten.

### Analytik

### Alterporriol I und J

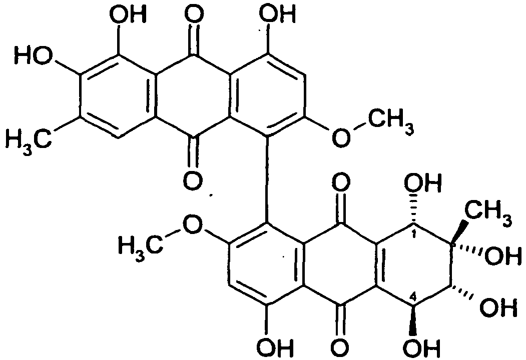

Aus dem HRESI-MS-Spektrum von Alterporriol I und J ergibt sich das Molekülion bei m/z 635,1406 [M+1]⁺, woraus die Summenformel C₃₂H₂₆O₁₄ und 20 % Unsättigung folgen. Die Daten der ¹H- und ¹³C-NMR-Spektren sowie des ESI/MS-Spektrums (m/z 634,9 [M+H]⁺) ähneln jenen der bekannten artverwandten Anthrachinone Altersolanol A und 6-O-Methylalaternin. Die Art der Bindung zwischen den beiden Anthrachinon-Einheiten ergibt sich aus Interpretation der COSY- und HMBC-Spektren und Vergleich mit NMR-Daten der Verbindungen Altersolanol A und 6-O-Methylalaternin. Das in nachstehender Tabelle 1 angegebene ¹H-NMR-Spektrum zeigt Signale von zwei tertiären Methylgruppen bei δ 1,110 (CH₃-7') und 1,114 (CH₃-7'*), zwei aromatischen Methylgruppen bei δ 2,17 (CH₃-2) und δ 2,16 (CH₃-2*), und vier Methoxygruppen, die teilweise überlappen und bei δ 3,68, 3,70 und 3,71 (aus dem Integral abgeleitet) schwingen. Zusätzlich sind überlappende Singuletts bei δ 4,04 (H-8'/H-8'*), überlappende Dubletts bei δ 4,45 (H-5'/H-5'*) und überlappende Dubletts bei δ 3,54 (H-6'/H-6'*) zu beobachten. Der aromatische Bereich zeigt fünf Protonenresonanzen bei δ 7,40, 6,94, 6,93, 6,91 und 6,90. Weiters sind im ¹H-NMR von Alterporriol keine meta-koppelnden Protonen zu erkennen. Das COSY-Spektrum zeigt jedoch klare Fernkorrelationen zwischen CH₃-2 und H-1, was belegt, dass die 7-O-Methylalaternin-Gruppierung entweder bei C-6 oder C-8 an die Altersolanol A-Gruppierung gebunden ist. Die Interpretation des ebenfalls in Tabelle 1 angeführten HMBC-Spektrums ergibt, dass das bei δ 6,93 / δ 6,94 beobachtete aromatische Proton (H-6/H-6*) eine starke Korrelation mit zwei sauerstofftragenden Kohlenstoffatomen bei δ 165,1 und 165,6 (C-5 und C-7) aufweist. Das bei δ 6,90 / δ 6,91 schwingende aromatische Proton (H-3'/H-3'*) korreliert mit zwei sauerstofftragenden Kohlenstoffatomen bei δ 164,2 und 164,3 (C-2' und C-4'), was C-8 als Verbindungsstelle zwischen den beiden Monomeren anzeigt.

Weiters zeigt die Interpretation des HMBC-Spektrums in Tabelle 1, dass H-1 mit C-9 (δ 180,4), C-4a (δ 129,0) und C-3 (δ 150,9) korreliert, während H-6/6* mit C-8 (δ 123,0) und C-10a (δ 109,3) korreliert. Darüber hinaus wurden starke Korrelationen für H-3' mit den Kohlenstoffatomen C-2', C-4' (δ 164,2, 164,3), C-1' (δ 123,1), C-4'a (δ 109,7) und der Ketogruppe an C-10' (δ 188,8) festgestellt, letztere aufgrund von W-Kopplung.

Zur Bestimmung der relativen Konfiguration von Alterporriol I und J im aliphatischen Ring wurde eine ROESY-Messung durchgeführt. Das Signal von CH₃-7' zeigt hierbei starke Korrelationen mit H-6', H-8', OH-6' und OH-8', was anzeigt, dass CH₃-7' äquatorial vorliegt. Weiters korreliert Proton H-8' mit Proton H-6', wobei Letzteres in diaxialer Beziehung mit H-5' steht (was aus den Kopplungskonstanten abgeleitet werden kann). Auf diese Weise wurden die Verbindungen Alterporriol I und Alterporriol J als neue Naturprodukte identifiziert.

**Tabelle 1: ¹H-NMR- und ¹³C-NMR-Daten von Alterporriol I und J**

| **Atome** | **¹H** | **¹³C** | **HMBC** |
|---|---|---|---|
| 1 | 7,30 (s) | 122,5 | 3, 9, 2-Me, 4a |
| 2 | | 130,8 | |
| 3 | | 150,9 | |
| 4 | | 160,1 | |
| 4a | | 129,0 | |
| 5 | | 165,6^{*} | |
| 6 | 6,93 (s) / 6,94 (s) | 103,8 | 5, 7, 8, 10a |
| 7 | | 165,1* | |
| 8 | | 123,0 | |
| 8a | | 130,82 | |
| 9 | | 180,45 | |
| 9a | | 128,9 | |
| 10 | | 190,3 | |
| 10a | | 109,3 | |
| 1' | | 123,1 | |
| 2' | | 164,2# | |
| 3' | 6,90 (s) / 6.91 (s) | 103,8 | 1', 2', 4', 4'a, 10' |
| 4' | | 164,3# | |
| 4'a | | 109,7 | |
| 5' | 4,45 (d: 7,25) | 68,5 | 6', 8'a, 10'a |
| 6' | 3,54 (d: 7,25) | 73,7 | 5', 8' |
| 7' | | 72,8 | |
| 8' | 4,04 (s) | 68,3 | 6', 7', 8'a, 9', 10'a, 2-Me |
| 8'a | | 142,52 | |
| 9' | | 183,9 | |
| 9'a | | 123,5 | |
| 10' | | 188,8 | |
| 10'a | | 143,5 | |
| 2-Me | 2,164 / 2,175 | 16,4 / 16,1 | 1, 2, 3, 4 |
| 7'-Me | 1,110 / 1,114 | 22,24 | 6', 7', 8' |
| 7-OMe | 3,68 / 3,70 | 56,9 / 56,8 | 7 |
| 2'-OMe | 3,71 | 56,7 | 2' |
| OH (4 & 4') | 13,05 & 13,66 | | |
| OH | 4,84 | | |
| | 5,00 | | |
| | 5,46 | | |

### 3-O-Acetyl-Altersolanol M

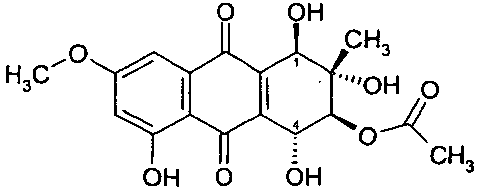

3-O-Acetyl-Altersolanol M wurde in Form von rotbraunen Kristallen isoliert. Das HRESI-MS-Spektrum zeigt einen Hauptpeak bei m/z 379 ([M+H]⁺), was 42 Masseneinheiten (d.h. eine Acetylgruppe) mehr als bei Altersolanol A sind, woraus die Summenformel C₁₈H₁₈O₉ folgt. Das ¹H-NMR-Spektrum enthält drei austauschbare alkoholische Hydroxylgruppen, zwei Dubletts bei δ 5,34 und 5,98 sowie ein Singulett bei 4,86, die 4-OH, 1-OH bzw. 2-OH zugeordnet werden können. Außerdem sind zwei Singuletts bei δ 1,13 und 2,11 (aus dem Integral abgeleitet) zu beobachten, die zwei Methylgruppen entsprechen. Die *meta*-koppelnden Protonen schwingen bei δ 6,85 (H-6) und δ 7,04 (H-8), während die aromatische Methoxygruppe bei δ 3,91 liegt. Im COSY-Spektrum, korreliert das Hochfeld-Dublett (1-OH) mit dem aliphatischen Niedrigfeld-Proton H-1, außerdem erscheint das Proton H-4 bei δ 4,68, das mit der Hydroxylgruppe (4-OH) am Kohlenstoffatomen C4 und mit dem Proton H-3 korreliert. Das gesamte aliphatische Spinsystem drückt auf H-1, H-3 und H-4, was zusammen mit den entsprechenden Hydroxylfunktionalitäten klar zeigt, dass die Acetylgruppe an Position C3 liegt. Weiters stützen im HMBC-Spektrum die den Protonen, d.h. H-1, H-3 und H-4 zuzuschreibenden Korrelationen (mit 1, 2, 9, 9a, 2-Me) sowie die Korrelationen der aromatischen Protonen vollkommen die oben angegebene Struktur von 3-O-Acetyl-Altersolanol M. Die relative Stereochemie von 3-O-Acetyl-Altersolanol M wurde aus den im ¹H-NMR-Spektrum beobachteten Kopplungskonstanten sowie aus den im ROESY-Spektrum detektierten Korrelationen abgeleitet. Der hohe Wert von J₁₋₂ (7,52 Hz) zeigt, dass H-4 und H-3 in diaxialer Beziehung stehen, was sich im ROESY-Spektrum bestätigt. Zudem zeigt die Methylgruppe bei δ 1,13 Korrelationen mit H-1, 1-OH, 2-OH und H-3, die sich durch ihre äquatoriale Lage erklären lassen. Schließlich zeigt Proton H-3 eine deutliche Korrelation mit Proton H-1, was die relative Konfiguration des aliphatischen Rings anzeigt.

**Tabelle 2: ¹H-NMR- und ¹³C-NMR-Daten von 3-O-Acetyl-Altersolanol M**

| **Atome** | **¹H** | **¹³C** | **HMBC** |
|---|---|---|---|
| 1 | 4,37 d (5,94) | 68,61 | 1, 2, 9a, 9, 2-Me |
| 2 | | 72,22 | |
| 3 | 5,25 d (7,62) | 76,75 | 4,11 |
| 4 | 4,68 dd (6,23, 7,59) | 65,92 | 4a |
| 4a | | 143,84 | |
| 5 | | 163,20 | |
| 6 | 6,85 d (2,52) | 106,07 | 7, 8, 4a, 5 |
| 7 | | 165,55 | |
| 8 | 7,04 d (2,49) | 106,80 | 4a, 6, 7, 9 |
| 8a | | 133,24 | |
| 9 | | 183,36 | |
| 9a | | 141,99 | |
| 10 | | 187,78 | |
| 10a | | 109,55 | |
| 11 | | 170,20 | |
| 2-Me | 1,13 | 21,76 | 1 |
| 11-Me | 2,11 | 20,91 | 11 |
| 6-OMe | 3,91 | 56,32 | 7 |
| 1-OH | 5,98 d (5,97) | | |
| 2-OH | 4,86 s | | 1 |
| 4-OH | 5,34 d (6,18) | | |

### Aktivitätsbestimmung - antimikrobielle/antifungale Wirkung

Die Aktivität der neuen Verbindungen wurden in zwei unterschiedlichen Screeningsystemen getestet. Die antibakterielle und antifungale Aktivität wurde in einem MHK-Test untersucht. MHK steht für die "minimale Hemm-Konzentration" (engl.: MIC für "minimal inhibitory concentration") und bezeichnet die niedrigste Konzentration einer Substanz, bei der mit bloßem Auge keine Vermehrung von Mikroorganismen wahrgenommen werden kann. Bestimmt wird die MHK mit einem so genannten Titerverfahren, bei dem die Substanz ausverdünnt und anschließend der Erreger zugefügt wird.

In der Regel wird so die Konzentration eines Antibiotikums bestimmt, die das Wachstum eines Bakterienstammes gerade noch hemmt. Die MHK wird in Mikrogramm pro Milliliter µg/ml) angegeben, und die Verdünnungen erfolgen in der Regel in log2-Schritten. Hierin wurde eine Ausgangskonzentration von 250 µg/ml jeweils auf das Doppelte verdünnt, was folglich Testkonzentrationen von 250 µg/ml, 125 µg/ml, 62,5 µg/ml, 31,2 µg/ml, 15,6 µg/ml, 7,8 µg/ml usw. ergab. Niedrigere Werte spiegeln demnach bessere Aktivität als Antiinfektivum wider.

Die Tests wurden nach den vom EUCAST (European Committee for Antimicrobial Susceptibility Testing) geforderten Standards und gemäß den AFST- ("Antifungal Susceptibility Testing") Vorschriften der European Society of Clinical Microbiology and Infectious Diseases (ESCMID) durchgeführt.

Das Screeningsystem für Viren ist ein Infektionssystem, bei dem Wirtszellen *in vitro* infiziert werden und die Testsubstanz vor oder nach der Infektion zugesetzt und ihre Aktivität bestimmt wird. Alle diese Tests wurden gemäß den betriebsinternen Standardvorschriften von SeaLife Pharma zum Arzneimittelscreening durchgeführt, wobei analoge Verdünnungsreihen wie im antibakteriellen/antifungalen Test eingesetzt wurden.

In der umseitigen Tabelle 3 sind die Testergebnisse bezüglich der antiinfektiven Wirkung gegen einige multiresistente Bakterien und Pilze (die allesamt freundlicherweise von Prof. Georgopulos von der Medizinischen Universität Wien zur Verfügung gestellt worden waren) für 3-O-Acetyl-Altersolanol M, Alterporriol I und J sowie einige bekannte und strukturell ähnliche Vergleichssubstanzen angeführt. Die Daten sind jeweils Mittelwerte von Mehrfachbestimmungen.

Es ist klar ersichtlich, dass die neuen Verbindungen der Erfindung ausgezeichnete Aktivität gegen vier Bakterienspezies, nämlich *Enterococcus faecalis* bzw. *faecium,* methicillinresistenten *Staphylococcus aureus, Streptococcus pneumoniae* und *Staphylococcus epidermis,* und darüber hinaus auch Wirkung gegen weitere Erreger zeigen, wobei 3-O-Acetyl-Altersolanol M in dieser Testreihe ein breiteres Aktivitätsspektrum aufweist als Alterporriol I und J.

**Tabelle 3: MHK-Werte bei multiresistenten Bakterien und Pilzen**

| **Substanzen** | **EC** | **KL** | **PS** | **EK** | **MRSA** | **STR** | **SE** | **ASP** |
|---|---|---|---|---|---|---|---|---|
| Beispiel 1: 3-O-Acetyl-Altersolanol M | 125 | 125 | 125 | 7,8 | 7,8 | 7,8 | 7,8 | 62,5 |
| Beispiel 2: Alterporriol I + J | | | | 7,8 | 7,8 | 7,8 | 7,8 | 125 |
| Bostrycin | | | | | 125 | 62,5 | 125 | |
| Altenusin | 62,5 | | | 62,5 | 31,2 | 31,2 | 31,2 | |
| Alterporriol A + B | | | | 15,6 | 15,6 | 7,8 | 15,6 | 62,5 |
| Altersolanol A | 62,5 | | | 15,6 | 7,8 | 7,8 | 7,8 | 7,8 |
| Altersolanol J | | 125 | | 31,2 | 62,5 | 15,6 | 62,5 | 125 |
| Ampelanol | | | | | | | | |
| Macrosporin | 62,5 | | | | 62,5 | | 62,5 | |
| Macrosporinsulfat | | | | | | 31,2 | | |
| Indolcarbaldehyd (Carboxaldehyd) | | | 125 | | | 62,5 | | |

| | | | | | | | | |
|---|---|---|---|---|---|---|---|---|
| EC *E. coli* KL *Klebsiella sp.* PS *Pseudomonas aeruginosa* EK *Enterococcus faecalis* bzw. *faecium* MRSA methicillinresistenter *Staphylococcus aureus* STR *Streptococcus pneumoniae* SE *Staphylococcus epidermis* AB *Acinetobacter baumanii* ASP *Aspergillus fumigatus* bzw. *faecalis* | | | | | | | | |

Als Ergebnis der Aktivitätstest der neuen Verbindungen der Erfindung gegen drei verschiedene Spezies respiratorischer Viren, nämlich ein humanes Rhinovirus (hrv), ein respiratorisches Synzytial-Virus (rsv) und Paraflu, die von der ATCC erhalten worden waren, wurde festgestellt, dass beide (bzw. alle drei) Verbindungen bereits ab einer Konzentration von 0,1 1 µg/ml für 100%igen Schutz der Zellen sorgten. Vorversuche mit Influenza- und Adenoviren haben ebenfalls bereits erste viel versprechende Ergebnisse geliefert.

### Aktivitätsbestimmung - zytotoxische Wirkung

Alterporriol I und J und vor allem 3-O-Acetyl-Altersolanol M wurden in mehreren Testreihen auf ihre Antikrebswirkung getestet. Die Testreihen waren sowohl zweidimensionale Standardtests als auch ein neu entwickeltes dreidimensionales Tumormodell.

### a) Alamar-Blue-Assay mit Melanomzellen

Bei diesem Test wird der Farbstoff Resazurin als Indikator zur Messung der Zytotoxizität von Substanzen verwendet. Dabei wird eine wässrige, blau gefärbte Resazurin-Lösung eingesetzt, die von normal funktionierenden Zellen (unter Verbrauch von NADH) allmählich zu Resorufin reduziert wird, das pinkfarben ist und fluoresziert. Zytotoxische Mittel verlangsamen oder stoppen diese Reduktion in Abhängigkeit von ihrer Toxizität.

Bei der Versuchsdurchführung wurden Melanomzellen mit mehreren Konzentrationen von 3-O-Acetyl-Altersolanol M, nämlich 1, 10 und 50 µg/ml, mit Altersolanol K und Alterporriol F als Vergleichssubstanzen sowie mit Alterporriol I + J, d.h. Verbindung (5) der vorliegenden Erfindung, in einer jeweiligen Konzentration von 10 µg/ml versetzt und 48 h lang inkubiert, wonach die Fluoreszenz der Proben spektralphotometrisch bei 570/600 nm gemessen wurde, die proportional zur Anzahl der überlebenden Zellen in den Proben ist.

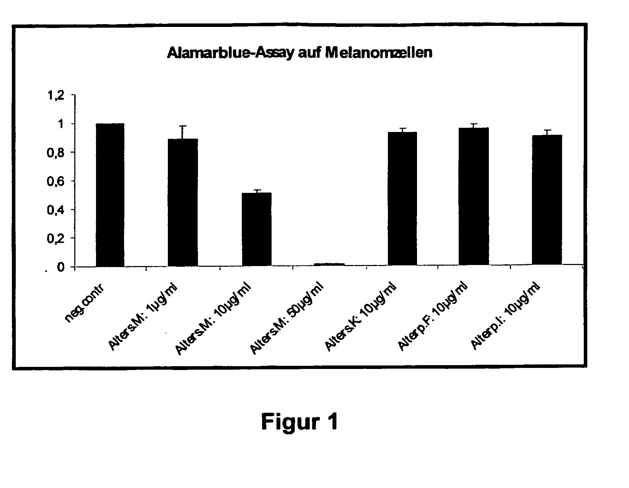

Die Messergebnisse wurden auf die Fluoreszenz einer Blindprobe ohne jegliches zytotoxisches Mittel bezogen und sind in Fig. 1 grafisch dargestellt. Von links nach rechts sind hier die Ergebnisse für den Blindvergleich ("neg.contr"), ansteigende Konzentrationen von 3-O-Acetyl-Altersolanol M (1, 10 und 50 µg/ml) sowie Altersolanol K, Alterporriol F und Alterporriol I + J aufgetragen.

Es ist deutlich zu erkennen, dass 3-O-Acetyl-Altersolanol M bereits in einer Konzentration von nur 1 µg/ml die Anzahl an überlebenden Zellen um mehr als 10 % hemmte. Diese Werte erreichten Altersolanol K, Alterporriol F und auch Alterporriol I/J erst bei einer Konzentration von 10 µg/ml, wobei das erfindungsgemäße Alterporriol I/J geringfügig besser abschnitt als die beiden Vergleichsbeispiele. Bei dieser Konzentration von 10 µg/ml verringerte 3-O-Acetyl-Altersolanol M die Anzahl an Melanomzellen freilich bereits um rund 50 %, und bei 50 µg 3-O-Acetyl-Altersolanol M pro ml waren kaum noch überlebende Zellen zu detektieren. Somit zeigten in diesem Test beide Verbindungen der vorliegenden Erfindung zytotoxische Aktivität, wobei 3-O-Acetyl-Altersolanol M jedoch zumindest die 10fache Wirkung zeigte.

Vergleichbare Ergebnisse (hierin nicht angeführt) wurden auch in einem "Electrical Cell-Substrate Impedance Sensing Technology"- (kurz ECIS-) Test erzielt, wobei 3-O-Acetyl-Altersolanol M erneut deutliche besser abschnitt als Alterporriol I/J.

### b) Dreidimensionaler Tumortest

Zusätzlich zum obigen zweidimensionalen Test wurde 3-O-Acetyl-Altersolanol M auch in einem innovativen dreidimensionalen Testsystem mit Melanomzellen auf seine zytotoxische Wirkung untersucht. Dabei werden kleine Tumoren in einem Matrixgel gezüchtet, in dem anschließend die Aktivität eines Antikrebsmittels durch Zusatz entsprechender Konzentrationen direkt getestet werden kann.

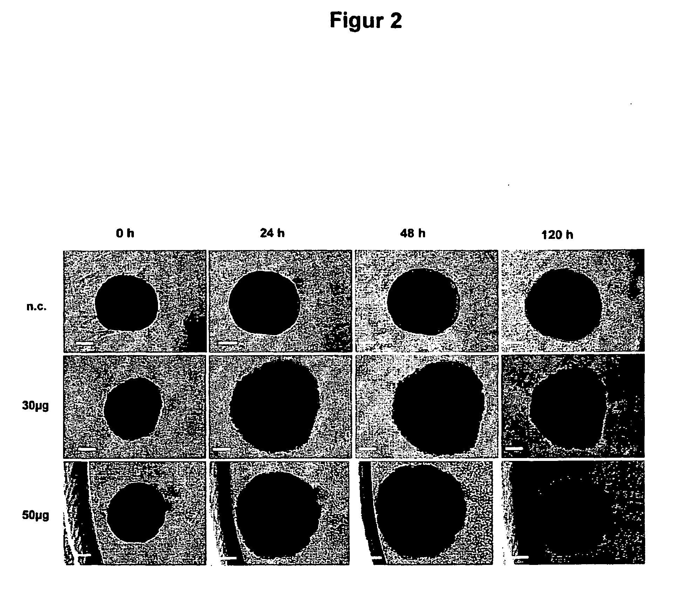

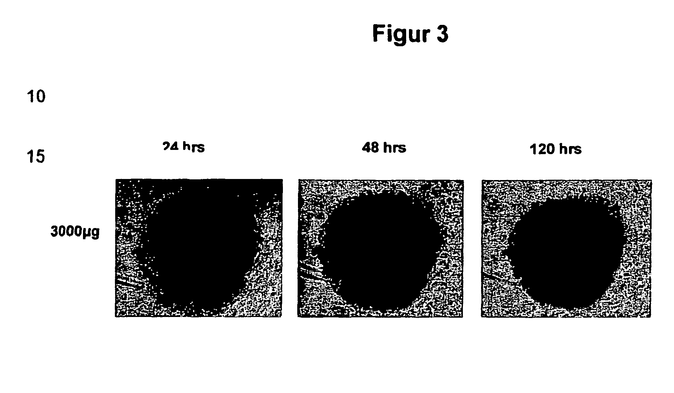

Ein Blindvergleich, d.h. Melanomzellen ohne zytotoxisches Mittel, sowie die gleiche Menge an Melanomzellen zusammen mit 30, 50 bzw. 3000 µg/ml an 3-O-Acetyl-Altersolanol M im Matrixgel wurden insgesamt 120 h lang bei 37 °C inkubiert. Von den Proben wurden nach 0, 24, 48 und 120 Stunden Phasenkontrastaufnahmen angefertigt, die in den Fig. 2 und 3 gezeigt werden.

Bei dem in der obersten Reihe in Fig. 2 gezeigten Blindvergleich ("n.c.") ist deutlich die Ausbildung einer äußeren Membran und die anschließende Melanineinlagerung in den kondensierten Tumor, bei stetiger Größenzunahme desselben, zu erkennen. Bei allen drei Konzentrationen von 3-O-Acetyl-Altersolanol M sind hingegen bereits nach 24 h sichtbare Degenerationserscheinungen der Tumoren sowie eine deutliche Größenabnahme nach 120 h festzustellen, wobei die zersetzende Wirkung von 3-O-Acetyl-Altersolanol M bei der in Fig. 3 dargestellten höchsten Konzentration, die das 100fache der niedrigsten Konzentration beträgt, erwartungsgemäß am klarsten zutage tritt.

Somit wurde eindeutig belegt, dass gemäß vorlegender Erfindung neue Verbindungen bereitgestellt werden, die sehr gute Wirksamkeit sowohl gegen mehrfach arzneimittelresistente bakterielle und fungale Erreger als auch gegen respiratorische Viren sowie gegen Krebszellen zeigen und daher breite Anwendung als Antiinfektiva und als Antikrebsmittel finden können. Speziell die Verbindung (4), 3-O-Acetyl-Altersolanol M, stellt einen viel versprechenden Wirkstoff auf allen genannten Anwendungsgebieten dar.

## Patentansprüche

1. (1*R*,2*S*,3*S*,4*R*)-3-Acetoxy-1,2,4,5-tetrahydroxy-7-methoxy-2-methyl-1,2,3,4-tetrahydroanthracen-9,10-dion (3-O-Acetyl-Altersolanol M):

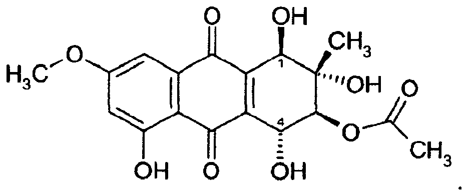

2. 8-(4,5,6-Trihydroxy-7-methyl-2-methoxy-9,10-dioxo-9H,10H-anthracen-1-yl)-(1*S*,2*S*,3*R*,4*S*)-1,2,3,4,5-pentahydroxy-7-methoxy-2-methyl-1,2,3,4-tetrahydro-9H,10H-anthracen-9,10-dion (Atropisomere Alterporriol I und J):

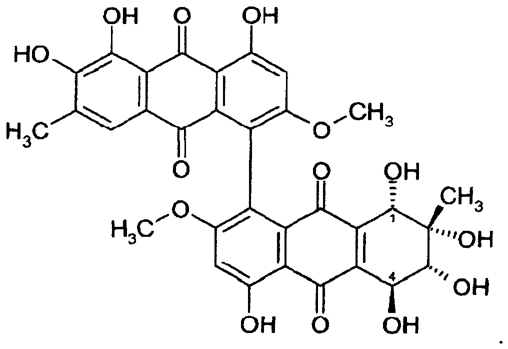

3. Verbindung nach Anspruch 1 oder 2 zur Verwendung als Antiinfektivum.

4. Verbindung nach Anspruch 1 oder 2 zur Verwendung nach Anspruch 3, **dadurch gekennzeichnet, dass** sie zur Verwendung als Antiinfektivum gegen grampositive und gramnegative Bakterien, Pilze und respiratorische Viren dient.

5. Verbindung nach Anspruch 1 oder 2 zur Verwendung nach Anspruch 4, **dadurch gekennzeichnet, dass** sie zur Verwendung als Antiinfektivum gegen mehrfach wirkstoffresistente ("multiple drug resistant", MDR-) Erreger dient.

6. Verbindung nach Anspruch 1 oder 2 zur Verwendung nach Anspruch 5, **dadurch gekennzeichnet, dass** sie zur Verwendung als Antiinfektivum gegen mehrfach wirkstoffresistente Stämme von methicillinresistentem *Staphylococcus aureus* (MRSA), *Staphylococcus epidermis, Streptococcus pneumoniae, Enterococcus faecalis* bzw. *faecium, Escherichia. coli, Klebsiella sp., Pseudomonas aeruginosa, Aspergillus sp.* sowie respiratorische Viren aus der Gruppe der humanen Rhinoviren und der respiratorischen Synzytial-Viren dient.

7. Verbindung nach Anspruch 1 oder 2 zur Verwendung als Antikrebsmittel.

8. Verfahren zur Herstellung einer Verbindung nach Anspruch 1 oder 2, **dadurch gekennzeichnet, dass** der Mikroorganismus *Stemphylium globuliferum* unter Wachstumsbedingungen fermentiert wird und die Verbindung, gegebenenfalls nach Zerstörung der Zellen des Mikroorganismus, aus der Kultur gewonnen wird.

9. Verfahren nach Anspruch 8, **dadurch gekennzeichnet, dass** die Verbindung durch Extraktion und anschließende Isolierung aus dem Rohextrakt gewonnen wird.

10. Verfahren zur Herstellung der Verbindung nach Anspruch 1, d.h. von 3-O-Acetyl-Altersolanol M, folgende Schritte umfassend:
a) die *syn*-Dihydroxylierung von (1*R*,2*R*)-1,2,5-Trihydroxy-7-methoxy-2-methyl-1,2-dihydroanthracen-9,10-dion (6) an C3 und C4 zu (1*R*,2*S*,3*R*,4*R*)-3-Acetoxy-1,2,4,5-tetrahydroxy-7-methoxy-2-methyl-1,2,3,4-tetrahydroanthracen-9,10-dion (7) 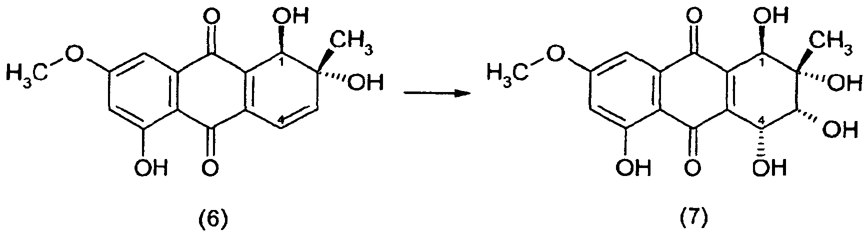
b) Überführung von (7) in das zyklische Sulfat (8) 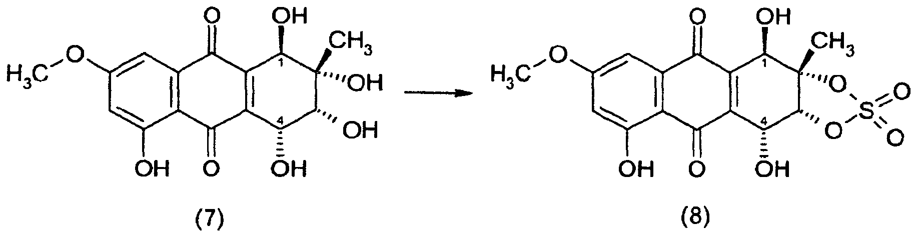
c) Acidolyse des zyklischen Sulfats (8) mit Essigsäure zu 3-O-Acetyl-Altersolanol M.

11. Verfahren zur Herstellung der Verbindung nach Anspruch 1, d.h. von 3-O-Acetyl-Altersolanol M, folgende Schritte umfassend:
a) Diels-Alder-Addition zwischen Naphthochinon (9) und Methylbutadien (10), worin die R unabhängig voneinander gleiche oder unterschiedliche Hydroxyl-Schutzgruppen darstellen, zum Tetrahydroanthrachinon (11) 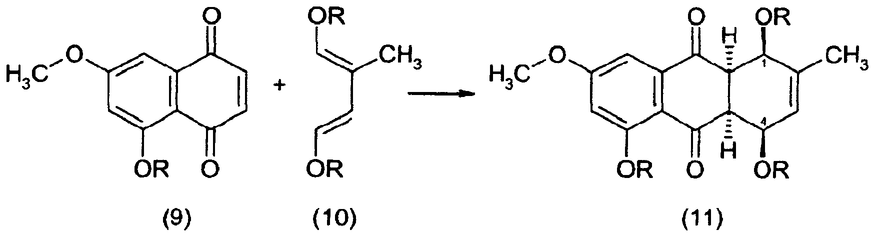
b) Oxidation der Doppelbindung zwischen C2 und C3 mit *meta*-Chlorperbenzoesäure, unter anschließender oder vorhergehender Mitsunobu-Reaktion zur Inversion der Sauerstoff-Funktionalität an C4, zum Epoxid (12) 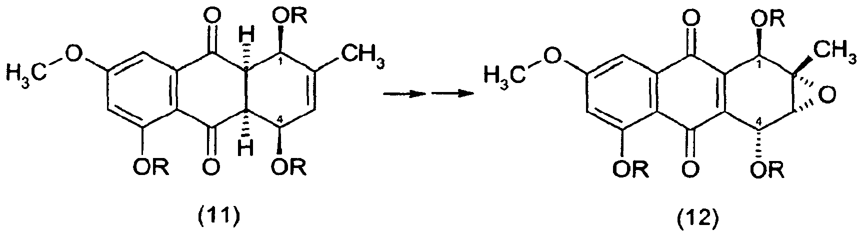
c) Acidolyse des Epoxids (12) mit Essigsäure, unter anschließender oder gleichzeitiger Abspaltung der Schutzgruppen R, zu 3-O-Acetyl-Altersolanol M.

12. Verfahren zur Herstellung der Verbindung nach Anspruch 1, d.h. von 3-0-Acetyl-Altersolanol M, folgende Schritte umfassend:
a) Diels-Alder-Addition zwischen Naphthochinon (9) und Methylbutadien (10), worin die R unabhängig voneinander gleiche oder unterschiedliche Hydroxyl-Schutzgruppen darstellen, zum Tetrahydroanthrachinon (11) 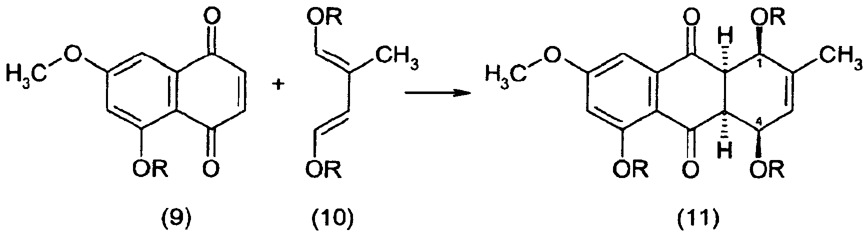
b) *syn*-Dihydroxylierung des Tetrahydroanthrachinons (11), unter anschließender oder vorhergehender Mitsunobu-Reaktion zur Inversion der Sauerstoff-Funktionalität an C4, zum Tetrahydroanthrachinon (13) 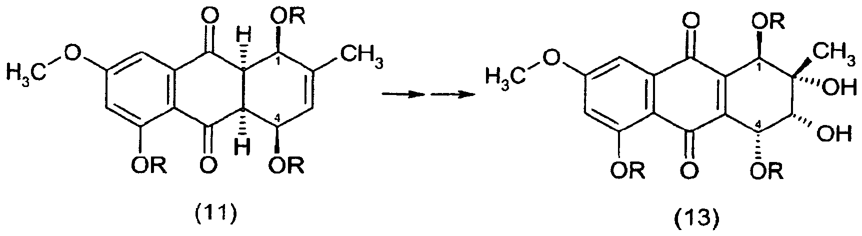
c) Überführung von (13) in das zyklische Sulfat (14) 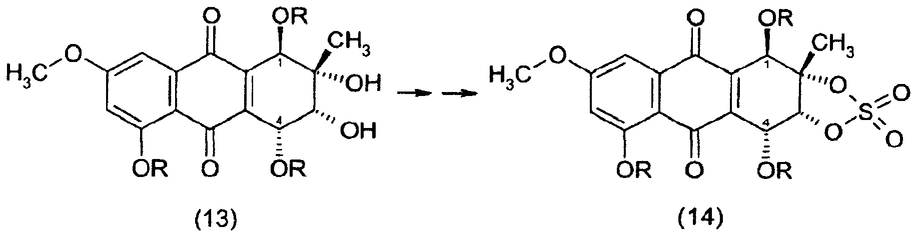
d) Acidolyse des zyklischen Sulfats (14) mit Essigsäure, unter anschließender oder gleichzeitiger Abspaltung der Schutzgruppen R, zu 3-O-Acetyl-Altersolanol M.

## Claims

1. (1*R*,2*S*,3*S*,4*R*)-3-Acetoxy-1,2,4,5-tetrahydroxy-7-methoxy-2-methyl-1,2,3,4-tetrahydroanthracene-9,10-dione (3-O-acetyl altersolanol M):

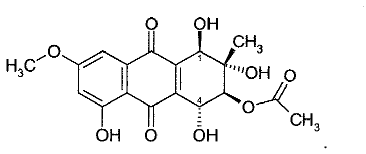

2. 8-(4,5,6-Trihydroxy-7-methyl-2-methoxy-9,10-dioxo-9H,10H-anthracen-1-yl)-(1 S,2S,3R,4S)-1,2,3,4,5-pentahydroxy-7-methoxy-2-methyl-1,2,3,4-tetrahydro-9H,10H-anthracene-9,10-dione (atropisomers alterporriol I and J):

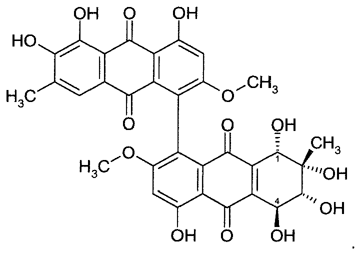

3. The compound according to claim 1 or claim 2 for use as an anti-infective.

4. The compound according to claim 1 or claim 2 for use according to claim 3, **characterized in that** said compound is for use as an anti-infective against Grampositive and Gram-negative bacteria, fungi, and respiratory viruses.

5. The compound according to claim 1 or claim 2 for use according to claim 4, **characterized in that** said compound is for use as an anti-infective against multiple drug resistant (MDR) pathogens.

6. The compound according to claim 1 or claim 2 for use according to claim 5, **characterized in that** said compound is for use as an anti-infective against multiple drug resistant strains of methicillin-resistant *Staphylococcus aureus* (MRSA), *Staphylococcus epidermis, Streptococcus pneumoniae, Enterococcus faecalis* or *faecium, Escherichia coli, Klebsiella sp., Pseudomonas aeruginosa, Aspergillus sp.* as well as respiratory viruses from the group of human rhinoviruses and respiratory syncytial viruses.

7. The compound according to claim 1 or claim 2 for use as an anti-cancer agent.

8. A method for producing the compound according to claim 1 or claim 2, **characterized in that** the microorganism *Stemphylium globuliferum* is fermented under growth conditions and the compound is obtained from the culture, optionally after having disrupted the cells of the microorganism.

9. The method according to claim 8, **characterized in that** the compound is obtained by extraction and subsequent isolation from the crude extract.

10. A method for producing the compound according to claim 1, i.e. 3-O-acetyl altersolanol M, comprising the following steps:
a) *syn*-dihydroxylating (1*R*,2*R*)-1,2,5-trihydroxy-7-methoxy-2-methyl-1,2-dihydroanthracene-9,10-dione (6) at C3 and C4 to obtain (1*R*,2*S*,3*R*,4*R*)-3-acetoxy-1,2,4,5-tetrahydroxy-7-methoxy-2-methyl-1,2,3,4-tetrahydroanthracene-9,10-dione (7) 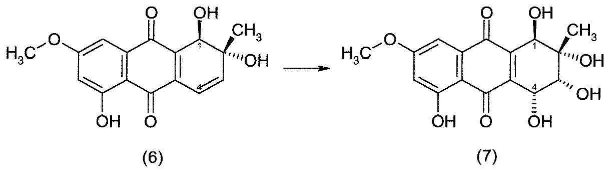
b) converting (7) into the cyclic sulfate (8) 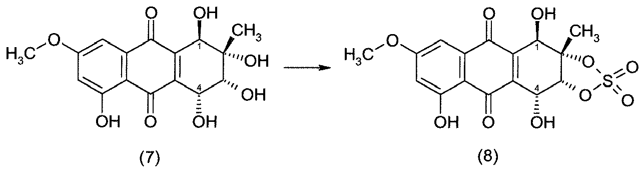
c) acidolyzing the cyclic sulfate (8) using acetic acid in order to obtain 3-O-acetyl altersolanol M.

11. A method for producing the compound according to claim 1, i.e. 3-O-acetyl altersolanol M, comprising the following steps:
a) Diels-Alder addition between naphthoquinone (9) and methylbutadiene (10), wherein the R independently represent identical or different hydroxyl protecting groups, in order to obtain tetrahydroanthraquinone (11) 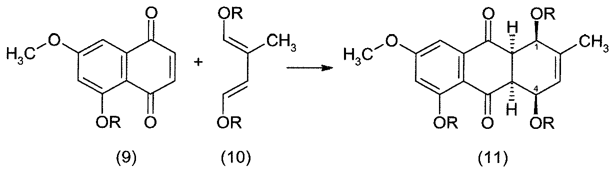
b) oxidation of the double bond between C2 and C3 using *meta*-chloroperbenzoic acid, carrying out a subsequent or precedent Mitsunobu reaction in order to invert the oxygen functionality at C4, in order to obtain the epoxide (12) 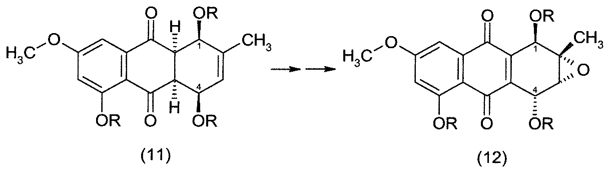
c) acidolysis of the epoxide (12) using acetic acid, subsequently or simultaneously cleaving the protecting groups R, in order to obtain 3-O-acetyl altersolanol M.

12. A method for producing the compound according to claim 1, i.e. 3-O-acetyl altersolanol M, comprising the following steps:
a) Diels-Alder addition between naphthoquinone (9) and methylbutadiene (10), wherein the R independently represent identical or different hydroxyl protecting groups, in order to obtain tetrahydroanthraquinone (11) 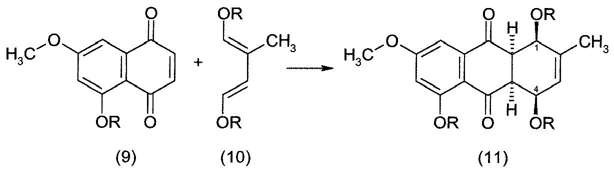
b) syn-dihydroxylation of the tetrahydroanthraquinone (11), carrying out a subsequent or precedent Mitsunobu reaction in order to invert the oxygen functionality at C4, in order to obtain the tetrahydroanthraquinone (13) 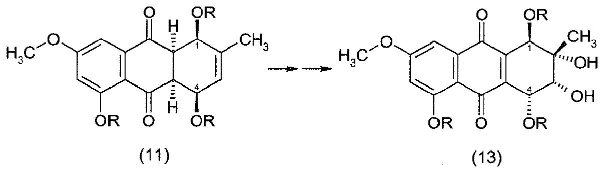
c) conversion of (13) into the cyclic sulfate (14) 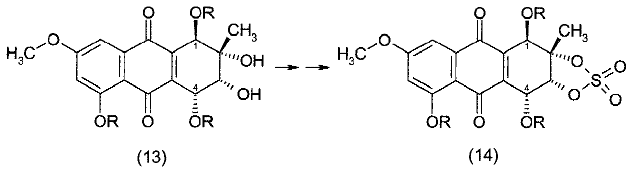
d) acidolysis of the cyclic sulfate (14) using acetic acid, subsequently or simultaneously cleaving the protecting groups R, in order to obtain 3-O-acetyl altersolanol M.

## Revendications

1. (1 R,2S,3S,4R)-3-Acétoxy-1,2,4,5-tétrahydroxy-7-méthoxy-2-méthyl-1,2,3,4-tétrahydroanthracène-9,10-dione (3-O-acétyle-altersolanol M):

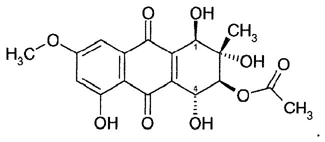

2. 8-(4,5,6-Trihydroxy-7-méthyl-2-méthoxy-9,10-dioxo-9H,10H-anthracène-1-yle)-(1*S*,2*S*,3*R*,4*S*)-1,2,3,4,5-pentahydroxy-7-méthoxy-2-méthyl-1,2,3,4-tétrahydro-9H,10H-anthracène-9,10-dione (atropisomères alterporriol I et J):

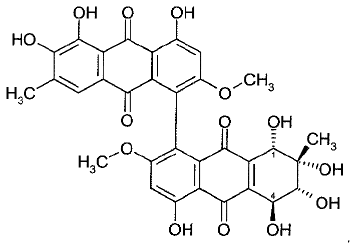

3. Composé selon la revendication 1 ou 2 pour utilisation comme anti-infectieux.

4. Composé selon la revendication 1 ou 2 pour utilisation selon la revendication 3, **caractérisé en ce que** ledit composé est pour utilisation comme anti-infectieux contre les bactéries Gram-positives et Gram-négatives, les champignons et les virus respiratoires.

5. Composé selon la revendication 1 ou 2 pour utilisation selon la revendication 4, **caractérisé en ce que** ledit composé est pour utilisation comme anti-infectieux contre les agents pathogènes résistants à de multiples médicaments.

6. Composé selon la revendication 1 ou 2 pour utilisation selon la revendication 5, **caractérisé en ce que** ledit compose est pour utilisation comme anti-infectieux contre des souches résistantes à de multiples médicaments de *Staphylococcus aureus* (MRSA) résistant à la méthicilline, de *Staphylococcus epidermis,* de *Streptococcus pneumoniae, d'Enterococcus faecalis* ou *faecium,* d*'Escherichia coli,* de *Klebsiella* sp., de *Pseudomonas aeruginosa, d'Aspergillus sp.* et contre des virus respiratoires du groupe des rhinovirus humaines et des virus respiratoires syncytiaux.

7. Composé selon la revendication 1 ou 2 pour utilisation comme agent anticancéreux.

8. Procédé pour la production d'un composé selon la revendication 1 ou 2, **caractérisé en ce que** le microorganisme *Stemphylium globuliferum* est fermenté dans des conditions de croissance et **en ce que** le composé est obtenu de la culture, éventuellement après avoir détruit les cellules du microorganisme.

9. Procédé selon la revendication 8, **caractérisé en ce que** le composé est obtenu par extraction et par l'isolation dudit composé de l'extrait brut.

10. Procédé pour la production d'un composé selon la revendication 1, c'est-à-dire de 2-O-acétyle altersolanol M, ledit procédé comprenant les étapes suivantes:
a) *syn-*dihydroxyler (1*R*,2*R*)-1,2,5-trihydroxy-7-méthoxy-2-méthyl-1,2-dihydroanthracène-9,10-dione (6) aux positions C3 et C4 pour obtenir (1R,2S,3R,4R)-3-acétoxy-1,2,4,5-tétrahydroxy-7-méthoxy-2-méthyl-1,2,3,4-tétrahydroanthracène-9,10-dione (7) 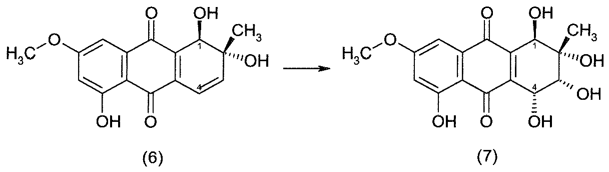
b) convertir (7) en sulfate cyclique (8) 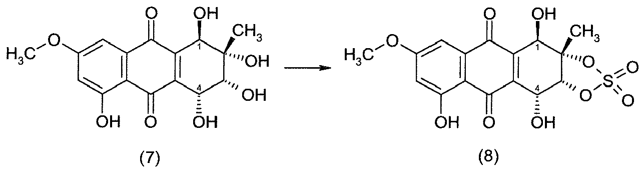
c) acidolyser ledit sulfate cyclique (8) en utilisant de l'acide acétique pour obtenir le 3-O-acétyle altersolanol M.

11. Procédé pour la production d'un composé selon la revendication 1, c'est-à-dire de 2-O-acétyle altersolanol M, ledit procédé comprenant les étapes suivantes:
a) addition de Diels-Alder entre naphthoquinone (9) et méthylbutadiène (10), dans lesquels les R représentent indépendamment des groupes hydroxyles protecteurs identiques ou différents, pour obtenir le tétrahydroanthraquinone (11) 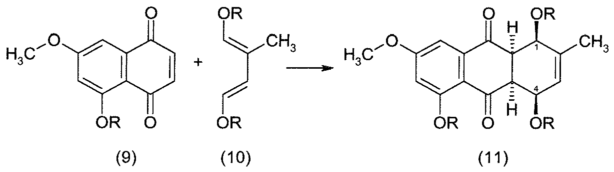
b) oxydation de la liaison double entre C2 et C3 en utilisant de l'acide *méta*chloroperbenzoïque, en effectuant une réaction de Mitsunobu après ou avant ladite oxydation pour invertir la fonctionnalité oxygénée à C4, pour obtenir l'époxyde (12) 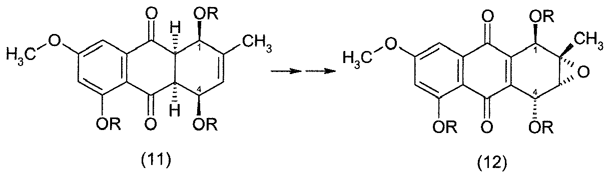
c) acidolyse de l'époxyde (12) en utilisant de l'acide acétique, en éliminant les groups protecteurs R par la suite ou en même temps, pour obtenir le3-O-acétyle altersolanol M.

12. Procédé pour la production d'un composé selon la revendication 1, c'est-à-dire de 2-O-acétyle altersolanol M, ledit procédé comprenant les étapes suivantes:
a) addition de Diels-Alder entre naphthoquinone (9) et méthylbutadiène (10), dans lesquels les R représentent indépendamment des groupes hydroxyles protecteurs identiques ou différents, pour obtenir le tétrahydroanthraquinone (11) 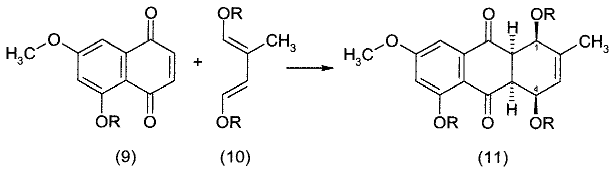
b) *syn*-dihydroxylation du tétrahydroanthraquinone (11), en effectuant une réaction de Mitsunobu après ou avant ladite *syn*-dihydroxylation pour invertir la fonctionnalité oxygénée à C4, pour obtenir le tétrahydroanthraquinone (13) 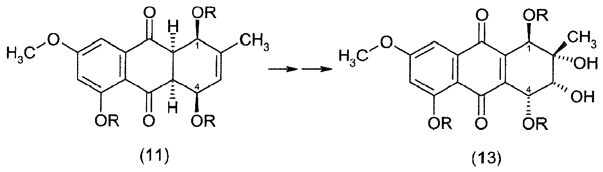
c) conversion de (13) en sulfate cyclique (14) 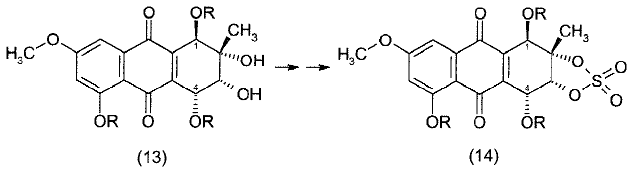
d) acidolyse du sulfate cyclique (14) en utilisant de l'acide acétique, en éliminant les groups protecteurs R par la suite ou en même temps, pour obtenir le3-O-acétyle altersolanol M.
